# FASCICULE DE BREVET EUROPEEN

(11) **EP 2 961 316 B1**
(45) Date de publication et mention de la délivrance du brevet: **28.11.2018**
(21) Numéro de dépôt: 14713205.4
(22) Date de dépôt: 03.03.2014
(51) Int. Cl.: A61B 5/055, A61B 5/11, A61B 5/113, A61B 6/00, G06T 7/00, G06T 7/20

(54) **PROCEDE AUTOMATIQUE DE DETERMINATION PREDICTIVE DE LA POSITION DE LA PEAU**
AUTOMATISCHES VERFAHREN ZUR VORABBESTIMMUNG DER POSITION DER HAUT
AUTOMATIC METHOD OF PREDICTIVE DETERMINATION OF THE POSITION OF THE SKIN

(30) Priorité: 01.03.2013 FR 1351855
(43) Date de publication de la demande: 06.01.2016
(73) Titulaire: Institut de Recherche sur les Cancers de l'Appareil Digestif IRCAD, 67000 Strasbourg (FR)
(72) Inventeur: SOLER, Luc, F-67202 Wolfisheim (FR); MARESCAUX, Jacques, F-67310 Scharrachbergheim (FR); NICOLAU, Stéphane, 77694 Kehl (DE); HOSTETTLER, Alexandre, F-67100 Strasbourg (FR)
(74) Mandataire: Nuss, Laurent
(86) Numéro de dépôt international: PCT/FR2014/050454
(87) Numéro de publication internationale: WO 2014/132008

(56) Documents cités:
- WO-A1-2010/113052
- WO-A2-2005/032647
- US-A1- 2009 010 514
- US-A1- 2009 175 406
- HOSTETTLER A ET AL: "A real-time predictive simulation of abdominal viscera positions during quiet free breathing", PROGRESS IN BIOPHYSICS AND MOLECULAR BIOLOGY, PERGAMON PRESS, OXFORD, GB, vol. 103, no. 2-3, 1 décembre 2010 (2010-12-01), pages 169-184, XP027546512, ISSN: 0079-6107 [extrait le 2010-09-29] cité dans la demande
- HOSTETTLER A ET AL: "Bulk modulus and volume variation measurement of the liver and the kidneys in vivo using abdominal kinetics during free breathing", COMPUTER METHODS AND PROGRAMS IN BIOMEDICINE, ELSEVIER, AMSTERDAM, NL, vol. 100, no. 2, 1 novembre 2010 (2010-11-01), pages 149-157, XP027287835, ISSN: 0169-2607 [extrait le 2010-09-15] cité dans la demande

## Description

La présente invention concerne le domaine des techniques d'imageries et de représentations exploratoires de sujets, plus particulièrement de sujets humains, notamment en temps-réel ou quasi temps-réel, et a pour objet un procédé automatique de détermination prédictive de la position de la peau d'un sujet, et des déformations/déplacements de cette dernière du fait de la respiration.

Actuellement, dans de nombreux contextes médicaux, les mouvements respiratoires constituent un paramètre qu'il est nécessaire de prendre en compte.

Par exemple, en radiothérapie, le volume cible planifié (PTV), qui n'est pas modifié durant le traitement, est volontairement surestimé, de manière à tenir compte des déplacements de la tumeur cible qui sont liés aux mouvements inconnus dus à la respiration. Si cela permet d'assurer une irradiation complète de la tumeur, cela augmente en même temps l'irradiation de tissus sains, ce qui n'est pas souhaitable pour la santé du patient.

Dans le contexte de ponctions percutanées en radiologie interventionnelle, pour atteindre sa cible, le radiologue demande au patient de bloquer sa respiration dans une position respiratoire similaire à celle de l'image médicale tomodensitométrique, IMR ou échographique utilisée pour repérer la cible. Par conséquent, la durée de l'opération est nettement plus longue que si la position de la tumeur était parfaitement connue.

Ces deux exemples illustratifs expliquent la nécessité de développer des méthodes précises de simulation du mouvement des organes induit par la respiration.

Le mouvement respiratoire a longtemps été considéré comme un mouvement parfaitement cyclique, et par conséquent, il a souvent été caractérisé par une seule variable représentant par exemple le volume d'air inspiré par le patient, ou encore un marqueur cutané positionné sur le thorax. Cette approche est bien connue par l'homme du métier dans le domaine considéré.

En pratique, ce n'est pas le cas, comme cela a notamment été exposé dans l'article "A real-time predicitive simulation of abdominal viscera positions during quiet free breathing" - "Simulation prédictive en temps-réel des positions des viscères abdominaux durant la respiration libre non forcée" - A. Hostettler et al., Progress in Biophysics and Molecular Biology, 103 (2010), p. 169-184, ELSEVIER. Dans cet article, il est prévu de suivre les mouvements de la peau, à l'aide d'un grand nombre de marqueurs optiques, ou de projection de lumière structurée, en particulier plusieurs marqueurs par plan axial d'intérêt, dans le but d'étudier ou simplement de connaître la position de la peau d'un patient, sans chercher à faire un modèle de déplacement de la peau.

En effet, dans cet article, les auteurs ont réalisé un suivi en temps-réel le mouvement de la peau au niveau du plan sagittal médian à l'aide de 8 marqueurs optiques (cf. section 2.2), avec mise en évidence que, comme le mouvement respiratoire est principalement dû à l'action combiné du diaphragme et des muscles intercostaux, dans le cadre de la respiration libre, l'action de ces deux muscles effecteurs n'a pas lieu en phase, et l'amplitude de leur mouvement respectif change au cours du temps. Par conséquent, il n'est pas possible de modéliser le mouvement respiratoire, ou celui d'une cible dans les viscères à l'aide d'une seule variable. Pour une même position d'un marqueur cutané, une cible localisée dans les viscères peut avoir plusieurs localisations significativement différentes.

Certaines de ces méthodes de simulation du mouvement des organes induit par la respiration tiennent compte du caractère non cyclique du mouvement respiratoire : la connaissance en temps-réel de la position de la peau du patient est alors obligatoire pour prédire le mouvement des organes.

En effet, la position de la peau peut être utilisée pour piloter un modèle numérique qui prédit la position des structures d'intérêt localisées dans la cavité abdominale, la cavité thoracique ou encore la paroi abdominale, comme par exemple dans l'article précité, dans lequel la surface antérieure complète de l'abdomen et du thorax est utilisée pour piloter le modèle, soit via l'utilisation de la lumière structurée, soit via une pluralité de marqueurs cutanés.

En outre, par le document "Bulk modulus and volume variation measurement of the liver and the kidneys in vivo using abdominal kinetics during free breathing" - A. Hostettler et al., Computer Methods and Programs in Biomedicine, 100 (2010), 149-157, on connaît, par ailleurs, un procédé de détermination des variations de position et de forme du foie et des reins durant une respiration libre d'un sujet humain, utilisant un modèle déformable de la forme du diaphragme pour suivre la peau du patient. Ce document reprend, pour l'essentiel, l'enseignement déjà divulgué dans le document précédemment cité.

S'il existe aujourd'hui des méthodes dites de "motion capture" permettant de fournir une modélisation temps-réel de la peau d'un sujet en mouvement, elles nécessitent cependant toutes des ressources matérielles importantes, rendant leur implémentation incompatible avec une mise en place au sein d'une salle d'opération (par exemple le produit connu sous la dénomination "alignRT" de la société ®visionRT). De plus, ces méthodes connues ne fournissent généralement qu'une estimation ou mesure d'une zone fractionnaire de la peau du patient et non pas de la totalité d'une zone d'intérêt (par exemple sur sensiblement 360° et sur une longueur importante dans la direction crânio-caudale).

Concrètement, cette information de surface est difficile à obtenir : durant la respiration, la peau se déplace presque partout et dans différentes directions, seule une partie de la surface qui est en contact avec la table reste immobile. De surcroît, dans de nombreuses applications, il est nécessaire de suivre la position de la peau avec une erreur ne devant pas dépasser l'ordre du millimètre.

Plus précisément, on connaît actuellement essentiellement trois méthodes différentes aptes à fournir un résultat exploitable, mais présentant toutes des inconvénients majeurs, à savoir :
a) Projection de lumière structurée, suivi par caméra et reconstruction de la surface de la peau :
   Dans ce cas, une projection de lumière structurée doit être effectuée à partir de plusieurs sources de manière à couvrir toute la surface de la peau du patient. Cela pose un problème de recouvrement à la jonction des multiples projections. Les caméras qui permettent de suivre les mouvements de la peau doivent également être nombreuses afin de garantir la visualisation complète de ces projections. Un algorithme de traitement d'image robuste doit être utilisé pour calculer en temps-réel la position de la peau qui est alors décrite comme un nuage de points plus ou moins ordonnés. Enfin, ce nuage de points doit le plus souvent être ré-échantillonné de manière à le rendre exploitable.
   Quoiqu'il en soit, le patient étant couché sur une table, cette méthode ne permet de localiser que la partie de la peau visible avec les caméras et sur laquelle est projetée la lumière structurée.
b) Suivi de marqueurs collés sur la peau du patient et calcul de la position de la peau par des méthodes d'interpolation :
   Cette méthode représente une simplification de la méthode précédente en réduisant de façon significative le nombre de points suivis. Bien que plus rapide, elle conserve les mêmes limites : les caméras doivent être suffisamment nombreuses pour suivre tous les marqueurs visibles. De ce fait, seule la partie de la peau visible aux caméras et sur laquelle sont placés les marqueurs pourra être suivie. Enfin, l'interpolation sera moins précise que la méthode précédente.
c) Suivi de marqueurs électromagnétiques collés sur la peau du patient (comme par exemple le système Aurora (nom déposé), développé par la société NDI) et calcul de la position de la peau par des méthodes d'interpolation :
   Dans ce cas, il n'y a pas besoin de caméra, mais les problèmes d'interpolation sont similaires. De surcroît, la précision de la mesure de la position fournie par les marqueurs électromagnétiques est plus faible que les méthodes précédentes (de 1 à 5 mm), l'erreur de mesure étant en plus potentiellement augmentée par la présence d'objets métalliques dans les champs de mesure.

Cette dernière limitation ajoutée à la limitation actuelle du nombre de marqueurs simultanément utilisables ainsi qu'à la fréquence limitée à 50Hz, rend de fait difficile la mise en oeuvre de cette méthode en routine clinique.

Compte tenu de ce qui précède, le but principal de l'invention est de proposer une solution de modélisation des mouvements de la peau d'un sujet humain qui fournisse une simulation tridimensionnelle fiable et précise d'au moins une zone d'intérêt, qui évolue en temps-réel, et-ne nécessite qu'un minimum de mesures et une puissance de traitement limitée **et met en oeuvre un modèle numérique déformable simple à obtenir et à utiliser.**

A cet effet, l'invention a pour objet un procédé automatique de détermination prédictive de la position et des déplacements de la peau d'un sujet humain ou animal dans une zone d'intérêt localisée au niveau du thorax et/ou de l'abdomen **présentant les caractéristiques de la revendication 1.**

Le principe de base de l'invention repose sur l'exploitation du constat surprenant fait par les inventeurs que, dans le cas d'un sujet humain respirant normalement, librement ou de manière assistée, une modélisation tridimensionnelle fiable et précise de la peau du sujet (au niveau de la région du thorax et de l'abdomen), permettant une prédiction en temps-réel des mouvements de la peau, pouvait être réalisée en ne relevant de manière continue que les informations relatives au déplacement et à la position d'un nombre restreint de points choisis de la peau, et en exploitant une modélisation du mouvement de la peau sur un nombre limité de plans axiaux consécutifs, créée à partir de données acquises précédemment.

Plus précisément, le repérage et le suivi en temps-réel de la position d'un unique point de la peau permet, en exploitant un modèle déformable créé à partir de plusieurs profils axiaux de la peau acquis à des positions respiratoires différentes, mais à une même position géographique, et dans un plan axial donné et comprenant ce point, de prédire la position et la conformation de ce même profil, elles-mêmes modifiées au cours du temps par les mouvements respiratoires au cours du temps, en fonction de la respiration du sujet.

L'invention sera mieux comprise, grâce à la description ci-après, qui se rapporte à deux modes de réalisation, donnés à titre d'exemples non limitatifs, et expliqués avec référence aux dessins schématiques annexés, dans lesquels :
les figures 1A à 1C sont des vues, obtenues par IRM, selon le plan sagittal médian d'un sujet humain pourvu d'un marqueur filaire selon l'invention, respectivement à l'état expiré (Fig. 1A), à l'état inspiré (Fig. 1B) et par superposition des différentes positions prises par le marqueur (par le biais de sa courbe médiane) dans les phases d'inspiration et d'expiration (Fig. 1C) ;
les figures 2A à 2D sont des vues, obtenues par IRM, selon un plan axial au niveau d'une zone d'intérêt d'un sujet humain pourvu du marqueur des figures 1, illustrant le profil de la peau déterminé à l'état expiré (Fig. 2A) et inspiré (Fig. 2B), le calcul de la courbe de référence à partir des deux courbes de profils extraites des figures 2A et 2B (Fig. 2C) et la génération d'un champ de déformation à partir des couples de points (appariement de points) appartenant aux deux courbes de profil de peau des figures 2A et 2B (Fig. 2D) ;
la figure 3 est une vue similaire à celle de la figure 1B avec une mise en évidence de la courbe médiane du marqueur ;
les figures 4A à 4C sont des vues de détail en coupe axiale du marqueur illustrant les étapes de détermination des contours et de l'inclinaison du marqueur ;
les figures 5A à 5D sont des vues en coupe axiale illustrant les étapes de segmentation et d'extraction du profil de peau ;
la figure 6 est une vue de détail en coupe axiale du marqueur et de son environnement immédiat, obtenue par superposition et illustrant différentes positions du centre de gravité du marqueur et l'hystérésis entre les positions prises durant l'inspiration et celles prises durant l'expiration ;
les figures 7A et 7B sont des représentations schématiques, respectivement de dessus et de côté, du tronc et de la tête du sujet humain équipé d'un marqueur selon l'invention pour le suivi des mouvements respiratoires, et,

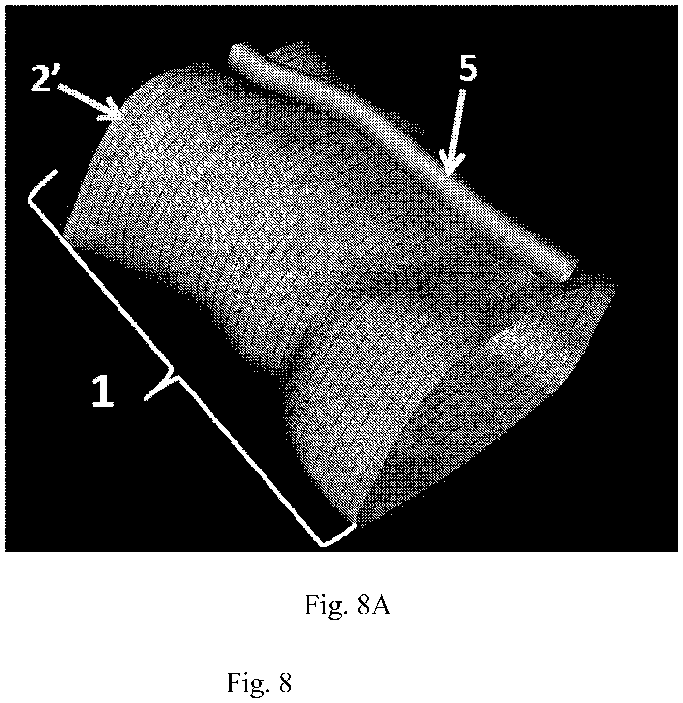

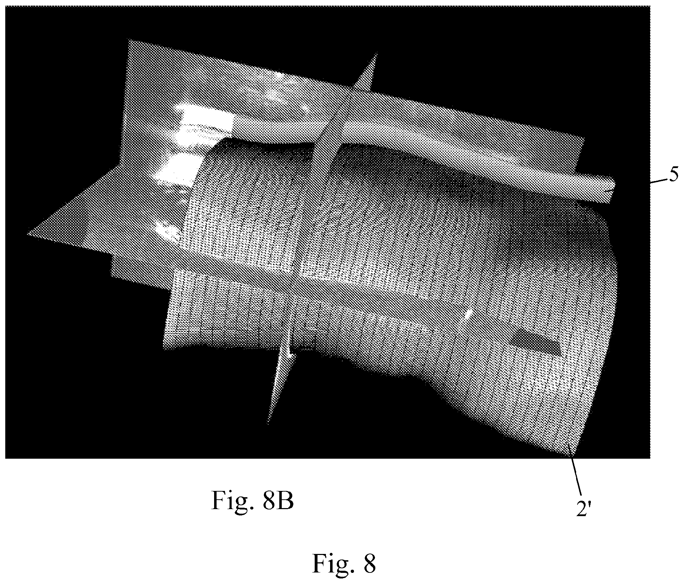

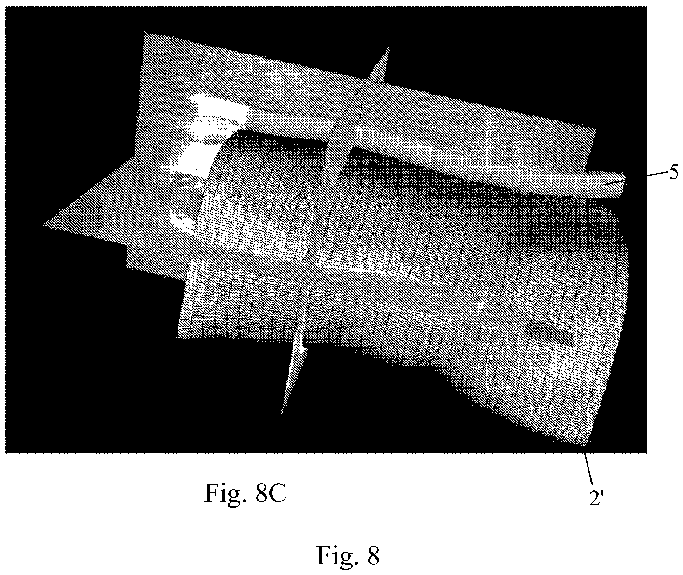

les figures 8A à 8C sont des représentations d'une simulation numérique tridimensionnelle de la zone d'intérêt d'un patient humain, asservie à la respiration, obtenue et utilisée dans le cadre de l'invention, les figures 8B et 8C montrant simultanément des vues acquises par l'imagerie dans des plans particuliers.

L'invention concerne un procédé automatique de détermination prédictive, par exemple sous la forme d'une simulation numérique, de la position et des déplacements de la peau 2 d'un sujet 1 humain ou animal dans une zone d'intérêt localisée au niveau du thorax et/ou de l'abdomen.

Dans le cadre d'une réalisation préférée de l'invention, on considère que le sujet 1 est allongé sur son dos et respire librement ou de manière assistée.

En accord avec les principes généraux et les caractéristiques essentielles de l'invention, le procédé consiste, de manière préalable, à acquérir ou déterminer plusieurs positions et configurations de la peau 2 dans au minimum deux positions du cycle respiratoire, de manière à pouvoir observer un déplacement respiratoire dont l'amplitude soit sensiblement semblable à l'amplitude correspondant à la respiration. Ces acquisitions sont effectuées à l'aide d'un imageur 3D classique de type CT-scan. En outre, ces acquisitions doivent couvrir la zone d'intérêt dont on souhaite ultérieurement pouvoir simuler le déplacement.

Une fois que ces images 3D ont été acquises, il convient d'en extraire la position de la peau, ce qui, par exemple en appliquant un simple opérateur de traitement d'image de type seuillage, est courant et peut être effectué sans difficulté pour l'homme du métier. Une fois que la position de la peau a été extraite et calculée dans chaque image 3D, on calcule les intersections de cette surface non plane avec un certain nombre de plans axiaux Pa choisis consécutivement le long de l'axe crânio-caudal du patient.

Il est possible de n'utiliser que deux acquisitions 3D, c'est-à-dire correspondant par exemple respectivement à la position expirée et à une position inspirée du patient, et on obtient alors une série de couple de courbes (représentant les profils de peau), correspondant respectivement au profil de la peau du patient en expiration et en inspiration dans chaque coupe axiale Pa.

L'invention prévoit de construire ensuite un modèle numérique indépendant pour chaque coupe axiale, correspondant, à titre d'exemple simple, à une interpolation linéaire entre la position de la courbe en expiration et la position de la courbe en inspiration.

Ainsi, en suivant de manière répétitive la position, d'un point ou d'une zone ponctuelle 4 spécifique de la peau 2 du sujet 1 au niveau de chacun des plans axiaux Pa précités, et dont la position est modifiée de manière significative durant le mouvement respiratoire, on peut fournir, sensiblement en temps réel, une simulation 3' du profil de la peau dans chaque plan axial Pa, en exploitant le modèle numérique déformable précédemment décrit. L'invention permet alors de créer une représentation tridimensionnelle 2' évolutive de la peau 2 au niveau de la zone d'intérêt, par interpolation entre la position simulée de la peau au niveau des différents plans axiaux Pa.

Une possible manière efficace de suivre un point pour chaque coupe axiale modélisée de la peau du patient (profil de peau) peut être de choisir de positionner tous les points axiaux à suivre sur un même plan, qui peut-être le plan sagittal médian. Dans ce cas, il suffit de suivre le déplacement du profil de la peau au niveau du plan sagittal médian pour connaitre la position de tous les points que l'on doit suivre dans chaque plan axial, puisque ces points constituent le profil de la peau au niveau du plan sagittal médian.

L'invention est décrite ci-après de manière plus détaillée, en relation avec un mode de réalisation particulier, illustré par les figures annexées et présentant des variantes pour certains aspects.

Comme précédemment, on considère que le sujet 1 est allongé sur son dos et respire librement ou de manière assistée.

Le procédé consiste, de manière préalable, à acquérir ou déterminer plusieurs positions et configurations du profil 3 de la peau 2 dans des plans axiaux Pa consécutifs prédéterminés, sensiblement perpendiculaires à l'axe crânio-caudal X et répartis le long de cet axe à des emplacements déterminés, ceci à des instants successifs donnés, dans au minimum deux positions du cycle respiratoire, de manière à pouvoir observer un déplacement respiratoire dont l'amplitude soit sensiblement semblable à l'amplitude correspondant à la respiration normale. La détermination de la position de la peau au niveau des profils axiaux peut être effectuée simultanément pour plusieurs profils à la fois, si le dispositif d'acquisition le permet.

Ce procédé consiste également, durant une phase préalable, pour chaque plan axial Pa considéré, à construire au moins un modèle numérique déformable de la peau (profil) à partir d'informations relatives aux différents profils de peau 3 acquis ou déterminés précédemment. On relève ensuite, de manière répétitive, la position courante réelle d'un point ou d'une zone ponctuelle 4 spécifique de la peau 2 du sujet 1 au niveau de chacun des plans axiaux Pa précité (préférentiellement repéré(e) à l'aide d'un marqueur 5 visible dans le procédé d'imagerie utilisé), dont la position est modifiée de manière significative par le mouvement respiratoire. Enfin, on fournit, sensiblement en temps réel, une simulation 3' du profil de la peau dans chaque plan axial Pa, en fonction de la position réelle relevée et en exploitant le modèle numérique déformable correspondant, mais également optionnellement d'une représentation tridimensionnelle 2' évolutive de la peau 2 au niveau de la zone d'intérêt, par interpolation entre les différents plans axiaux Pa.

De manière avantageuse, et afin de faciliter le suivi du déplacement de la peau et d'en augmenter la précision, il est souhaitable que les points ou zones ponctuelles 4 spécifiques de la peau 2, dont la position réelle est suivie dans le temps, soient disposé(e)s dans un plan parallèle proche ou confondu avec le plan sagittal médian PSM du sujet 1, en étant préférentiellement espacé(e)s mutuellement d'une distance de l'ordre du centimètre.

Toutefois, il peut aussi être prévu que les points ou zones ponctuelles 4 spécifiques, dont la position réelle est suivie dans le temps, sont réparti(e)s le long de l'axe crânio-caudal, en étant aligné(e)s ou non, leur nombre étant avantageusement au moins égal à environ 10 et leur espacement d'au plus 5 cm dans la direction de l'axe précité.

En accord avec l'invention, le procédé consiste préférentiellement, pour chaque plan axial Pa concerné, à générer sensiblement en temps-réel, une courbe 3' représentant le profil 3 de la peau 2 dans le plan axial Pa considéré, en appliquant à une courbe de base Cb de profil axial de la peau, une déformation, par exemple par déplacement d'au moins certains points espacés de la courbe de base, fonction de la position mesurée d'un point 4 de la peau 2 du sujet 1 situé dans le plan axial Pa considéré.

La courbe de base utilisée correspond préférentiellement au profil axial de la peau relevé à l'état d'expiration maximale du sujet 2.

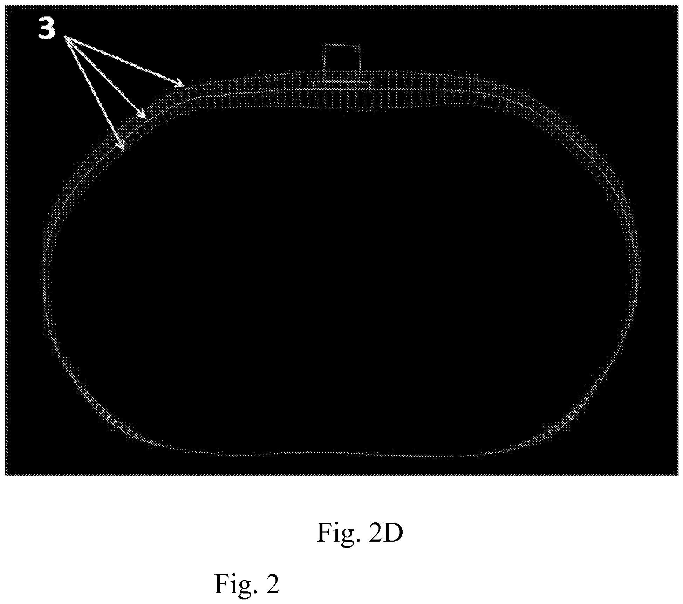

Comme l'illustre à titre d'exemple la figure 2D (pour des positions d'inspiration et d'expiration), chaque modèle numérique déformable de profil de peau, associé respectivement à l'un des plans axiaux Pa prédéterminés, est obtenu par appariement de points selon une direction sensiblement radiale, dans le plan axial Pa considéré, entre au moins deux conformations ou configurations sensiblement différentes de la courbe du profil de peau, correspondant à au moins deux états respectifs différents du mouvement respiratoire du sujet.

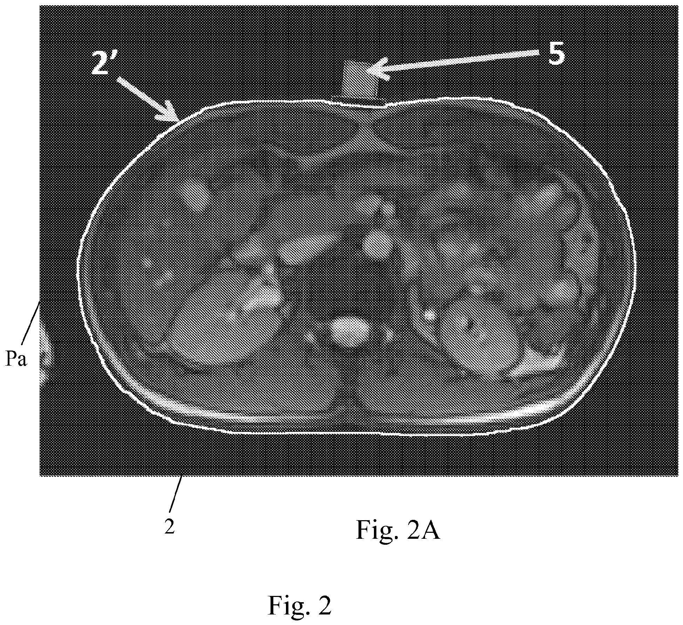

Conformément à une autre caractéristique de l'invention, précisant le mode de réalisation précédent et illustrée par les figures 2A à 2D, chaque modèle numérique déformable de profil de peau, associé à l'un des plans axiaux Pa prédéterminés, est obtenu par génération d'une courbe de référence Cr située à mi-distance entre les deux courbes de profils de peau correspondant aux états extrêmes d'inspiration et d'expiration, à déterminer les points d'intersection, avec les deux courbes précitées, d'une droite normale à la courbe de référence Cr et passant par un point de cette courbe, ce pour une pluralité de points espacés le long de cette dernière, et à rassembler de manière ordonnée ces couples de points d'intersection pour constituer un champ de déformation correspondant au déplacement de la peau 2 dans le plan axial Pa considéré entre les deux états extrêmes précités, en fonction de l'état respiratoire du sujet 1.

Les points précités de la courbe Cr sont suffisamment espacés pour que les droites normales ne se coupent pas avant d'intersecter les deux profils axiaux de la peau.

Comme indiqué précédemment, le modèle tridimensionnel de simulation globale de la peau est composé d'une pluralité de modèles axiaux élémentaires bidimensionnels et indépendants de la peau, établis dans des plans axiaux PA consécutifs le long de l'axe crânio-caudal.

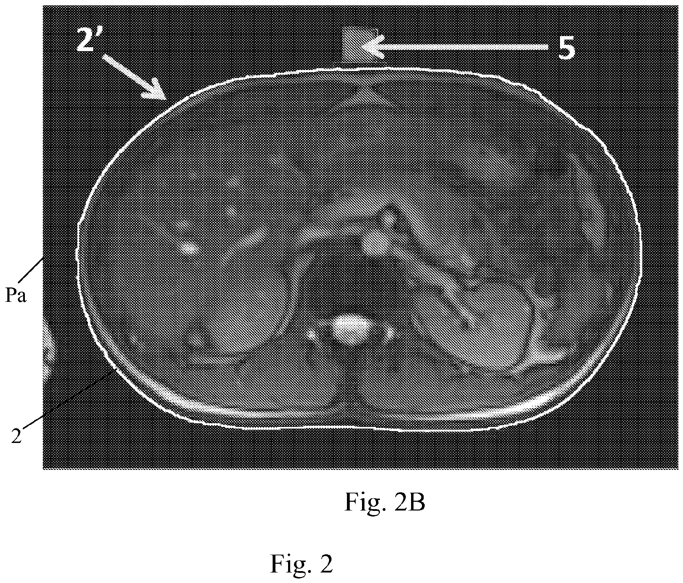

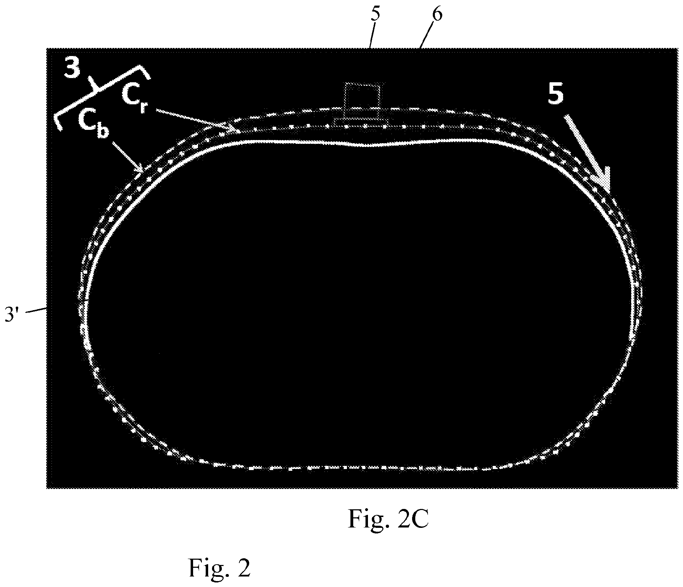

De manière pratique, une fois la peau segmentée dans les images correspondant respectivement aux positions extrêmes d'inspiration et d'expiration du sujet (Fig. 2A et 2B), chaque modèle axial peut être établi en exécutant les quatre étapes opératoires suivantes (voir Fig. 2C et 2D) :
1) Création d'une courbe de référence Cr située exactement à mi-distance entre les deux courbes correspondant aux deux positions extrêmes précitées. Cette étape peut être réalisée en utilisant une carte de distance correspondant à la différence entre le masque d'image relatif à la position de la peau à l'état inspiré et le masque d'image relative à la position de la peau à l'état expiré.
2) Ré-échantillonnage de la courbe de référence axiale Cr de manière à obtenir un ensemble de points consécutifs espacés de manière régulière.
3) Génération d'une droite normale à la courbe de référence Cr au niveau de chaque point de cette courbe ré-échantillonnée.
4) Détermination des deux points d'intersection correspondant à l'intersection de cette droite normale avec les courbes des états inspiré et expiré qui l'encadrent. On obtient alors un ensemble de vecteurs normaux à la courbe Cr qui représentent ainsi un champ de déformation reflétant le mouvement de la surface de la peau dans un plan axial Pa donné entre les deux positions originales des différents états respiratoires.

Ainsi, en répétant cette opération pour toutes les coupes axiales consécutives, on obtient un modèle axial du mouvement respiratoire tout le long de l'axe crânio-caudal, chaque ensemble d'informations résultant d'une acquisition 2D permet la construction d'un modèle axial local de la peau du patient. Compte tenu de la variation de la forme de la peau d'un sujet classique, la génération de modèles axiaux de la peau séparés entre eux d'environ 1 cm permet d'obtenir par interpolation un modèle tridimensionnel fidèle et précis de la peau du patient au niveau de la zone d'intérêt.

Afin de prendre en compte le phénomène d'hystérésis du mouvement de la peau durant la respiration, il est prévu dans le cadre du procédé selon l'invention de construire deux modèles numériques déformables de profil axiaux de la peau dans chaque plan axial Pa, à savoir un modèle pour les phases d'inspiration et un modèle pour les phases d'expiration, et à détecter le type de phase respiratoire en cours lors de l'élaboration des courbes simulées 3' de profil de peau dans les différents plans axiaux Pa, par l'intermédiaire d'un capteur spécifique ou en évaluant le sens de déplacement des points 4 en considérant individuellement leurs variations de position par rapport à leurs positions précédentes.

Selon une première variante de réalisation, les positions des points 4 spécifiques de la peau 2 sont relevés en temps réel au moyen d'un dispositif capteur optique, notamment à laser, avec mise en place préalable ou non de marqueurs optiques sur la peau 2 du patient aux emplacements des points 4 spécifiques, ces derniers étant avantageusement sélectionnés de manière à obtenir une modification significative de leur position spatiale durant le mouvement respiratoire du sujet.

Pour faciliter le suivi du profil sagittal de la peau qui correspond à la concaténation des différents points axiaux, des points 4 de la peau 2 dont les mouvements sont pris en compte dans le cadre du procédé en tant que paramètres d'entrée, les positions de ces points 4, situés dans le plan sagittal médian PSM du sujet 1 et dans les plans axiaux Pa prédéterminés sont déterminées par l'intermédiaire d'au moins un marqueur 5, préférentiellement un marqueur allongé continu, reposant directement ou indirectement sur la peau 2 du patient 1, épousant la surface locale de la peau 2 et disposé dans le plan sagittal médian PSM.

Alternativement, les marqueurs 5 mis en oeuvre consistent en des marqueurs de type électromagnétique, tels que par exemple des bobines miniatures, reposant directement ou indirectement sur la peau 2, le cas échéant avec un espacement connu par rapport à cette dernière.

Ainsi, les points 4 spécifiques de la peau 2 sont chacun repérés par l'intermédiaire d'un marqueur 5 particulièrement bien visible dans le procédé d'imagerie mis en oeuvre, ces points spécifiques 4 étant avantageusement localisés de telle manière que leur position spatiale est modifiée de manière significative durant le mouvement respiratoire du sujet.

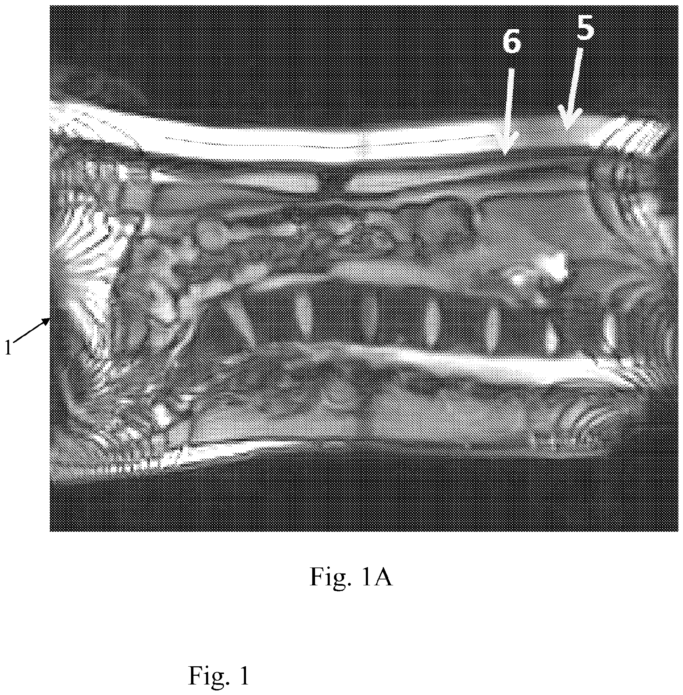

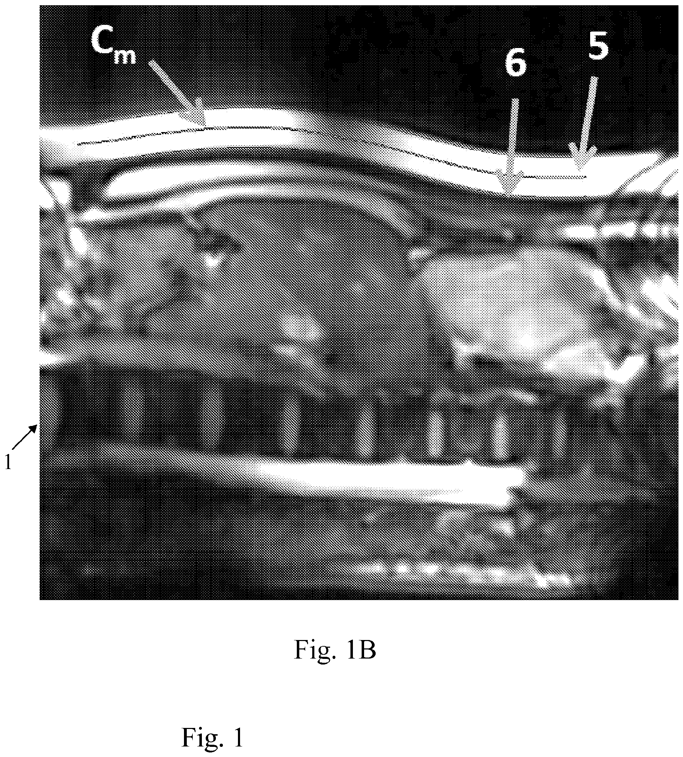

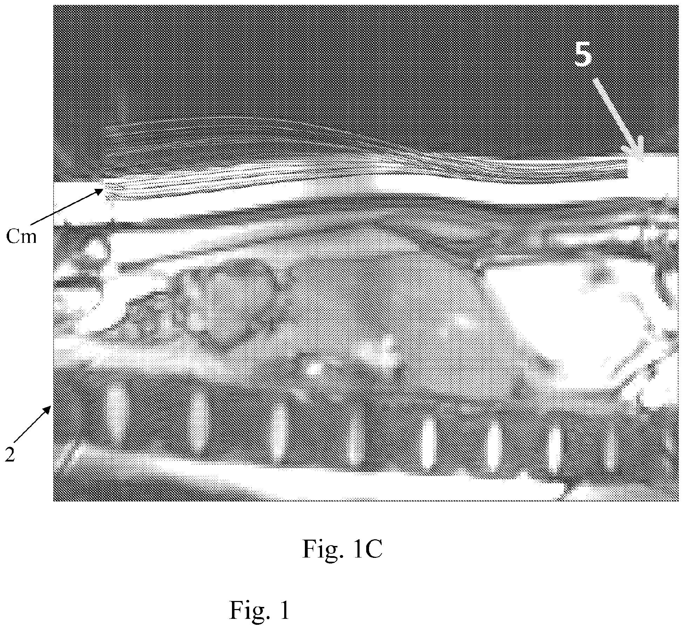

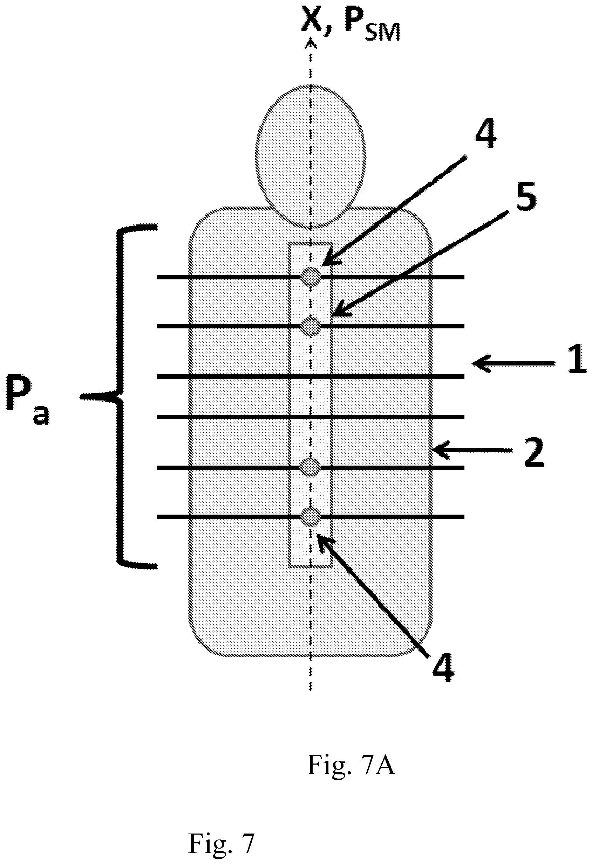

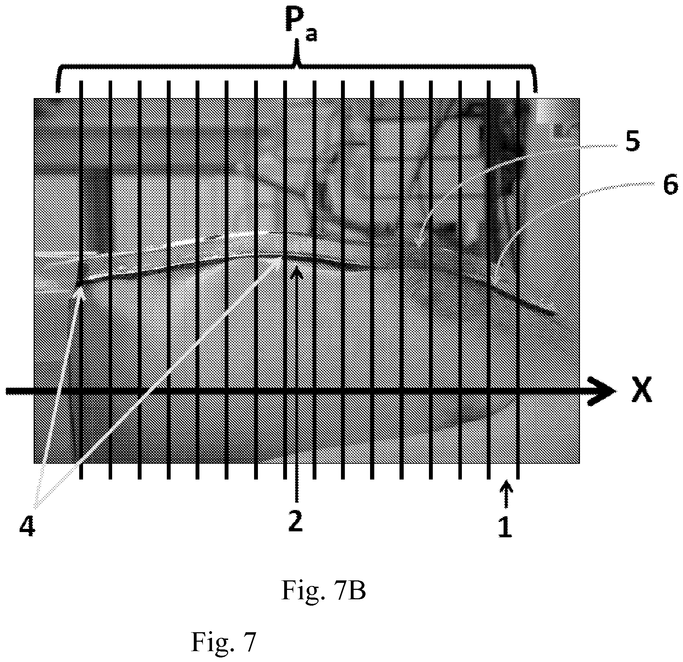

Comme le montrent les figures 1 à 4, 6 et 7, le marqueur 5 mis en oeuvre consiste en un corps souple et allongé, réalisé en un matériau particulièrement bien visible dans le système d'imagerie utilisé, s'étendant au moins sur la longueur de la zone d'intérêt dans la direction de l'axe crânio-caudal X, présentant une section de forme et de dimension minimale déterminées et situé à une distance déterminée de la peau 2 du sujet 1, en reposant sur une bande 6 d'un matériau souple et sensiblement non visible dans le système d'imagerie utilisé.

Connaissant les dimensions du marqueur 5 et celles de la bande de matériau 6 et en vue de faciliter le repérage fiable de la position du marqueur 5, le procédé peut avantageusement consister à déterminer et à relever, dans le plan sagittal, la courbe Cm correspondant à l'axe médian du marqueur 5 allongé, par exemple formé par la succession des différents centres de gravité en section dudit marqueur 5, et/ou dans les différents plans axiaux Pa concernés, les centres de gravité locaux dudit marqueur 5, et à déterminer les positions des points spécifiques 4 de la peau 2 du sujet 1 dont les mouvements sont pris en compte, en exploitant les informations relatives, d'une part, à cette courbe Cm ou à ces centres de gravité locaux, d'autre part, à la section et aux dimensions en section du marqueur 5 et, enfin, à l'épaisseur de la bande de matériau 6 intercalaire.

En accord avec une implémentation pratique préférée de l'invention, ressortant des figures annexées, il peut être prévu que, lorsque le procédé d'imagerie auquel est soumis le sujet 1 est basé sur la résonance magnétique, le marqueur allongé 5 est constitué par une barre formée d'eau gélifiée, avec une section carrée ou rectangulaire et une longueur suffisante pour couvrir au moins la zone du sujet dont on souhaite suivre les mouvements induits par la respiration dudit sujet 1 (zone d'intérêt).

Dans ce contexte, la segmentation de la peau dans des images sagittales obtenues par résonance magnétique est avantageusement réalisée en exploitant la très bonne visibilité du marqueur 5 dans les images IRM et son contraste par rapport à la bande textile 6 par l'intermédiaire de laquelle il repose sur la peau du sujet (les épaisseurs dudit marqueur et de ladite bande étant des données connues).

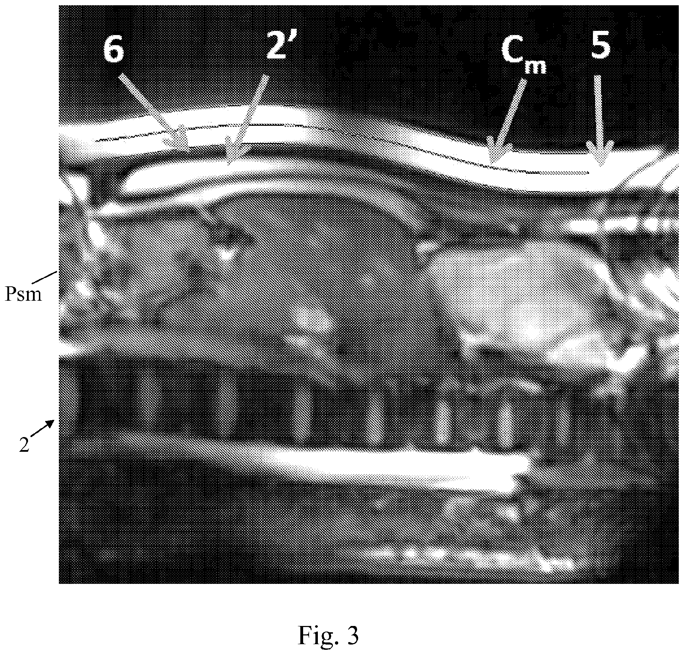

Plus précisément, cette phase de segmentation sagittale de la peau peut être décomposée en quatre étapes principales, à savoir :
1) Une image de travail 2D sur-échantillonnée et créée à partir de l'image 2D originelle (prise sur le sujet) en mettant en oeuvre une interpolation cubique (cette étape augmente la précision de la détermination de la position du marqueur 5). De manière pratique, et compte tenu des déformations et distorsions géométriques affectant les images IRM 2D (au niveau des deux côtés opposés des extrémités de la zone explorée - voir par exemple Fig. 3), seule la partie centrale des images initiales est exploitée (environ 60 %).
2) Compte tenu des différences de contraste entre des images 2D + t consécutives, il est avantageux de déterminer une valeur seuil correspondant à la position du marqueur 5. On recherche ensuite dans chaque droite verticale des différentes images la première suite de pixels consécutifs présentant une longueur au moins supérieure à environ 80 % de l'épaisseur du marqueur 5 et on calcule ensuite le centre du marqueur 5 le long de chaque droite verticale (permettant de déterminer la position du marqueur le long de cette droite). La meilleure valeur seuil correspond à celle qui minimise la distance moyenne entre la position simulée du marqueur et la position extraite par segmentation des images originelles.
3) On localise ensuite les parties du marqueur allongé 5 qui ne sont pas continues et on modifie localement le tracé de ce dernier en extrapolant les portions régulières du marqueur situé de part et d'autre de la partie discontinue.
4) Une opération de lissage des positions des centres de gravité consécutifs du marqueur est réalisée de manière à corriger les erreurs de segmentation dues à des manques locaux d'information (l'utilisation du centre de gravité permet de limiter l'influence de l'effet de volume partiel).

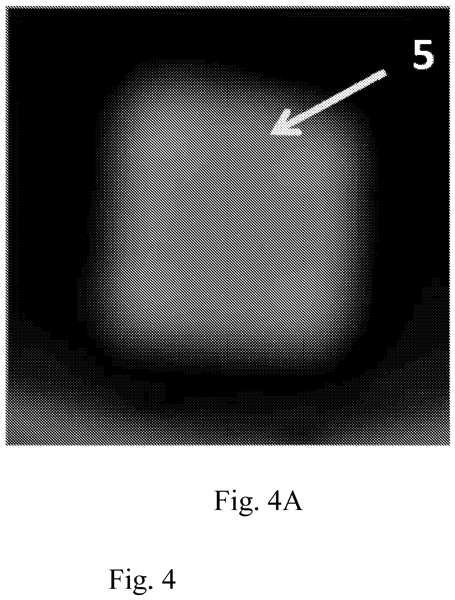

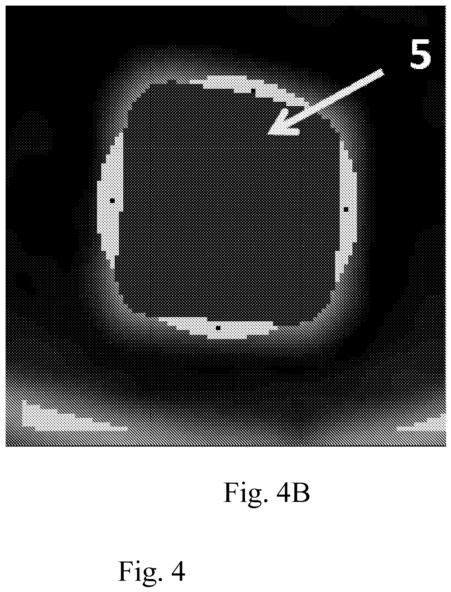

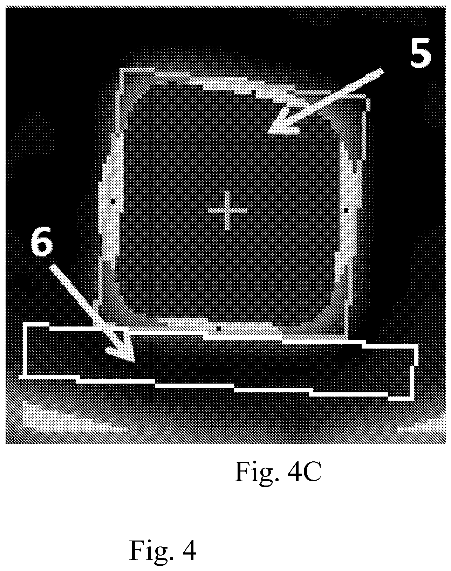

De même, la segmentation du marqueur 5 peut être réalisée dans les plans axiaux Pa ayant donné lieu à l'acquisition d'images 2D + t en exécutant les quatre étapes opératoires suivantes :
1) La recherche de la localisation du marqueur 5 à section carrée par exemple est limitée à une zone de recherche définie manuellement en début de procédure et ensuite centrée de manière automatique en se basant sur la position précédente du centre du marqueur.
2) On recherche la meilleure valeur seuil permettant d'obtenir au moins 80 % de la surface théorique de la section du marqueur (on recherche des pixels dont la valeur de gris est inférieure à la valeur seuil).
3) Dès que le masque représentant la section du marqueur est trouvé, on calcule son centre de gravité en utilisant un algorithme connu de calcul de barycentre.
4) On évalue ensuite l'inclinaison du marqueur à partir du masque précédent en effectuant par exemple les opérations suivantes : détermination des quatre portions de disque du plus grand disque centré sur le centre de gravité du marqueur (Fig. 4B) ; détermination des centres de gravité de ces quatre portions de disque (CG1, CG2, CG3, CG4) ; détermination des inclinaisons horizontale et verticale du marqueur par approximation à partir des segments reliant, d'une part, les points CG1 et CG3 et, d'autre part, les points CG2 et CG4.

Enfin, la segmentation de la peau dans les images axiales 2D + t est réalisée en utilisant les informations relatives à la position du marqueur 5 fourni précédemment.

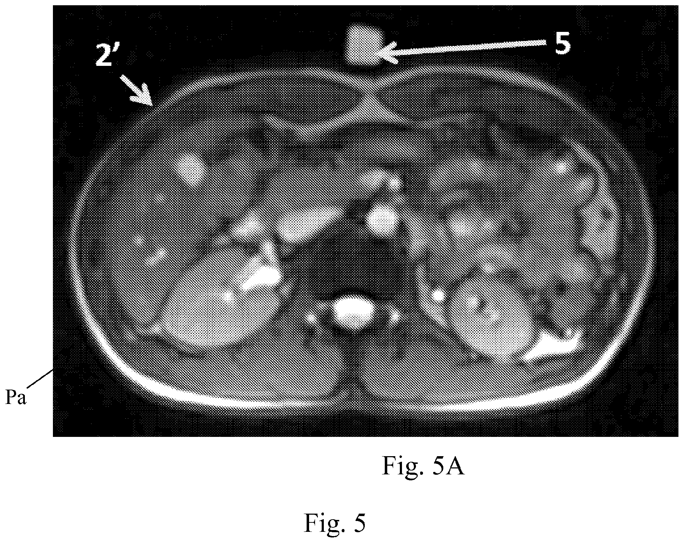

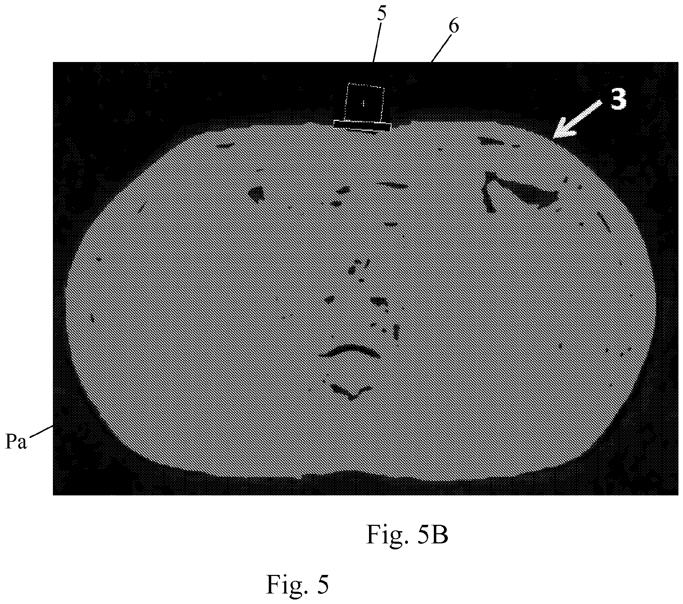

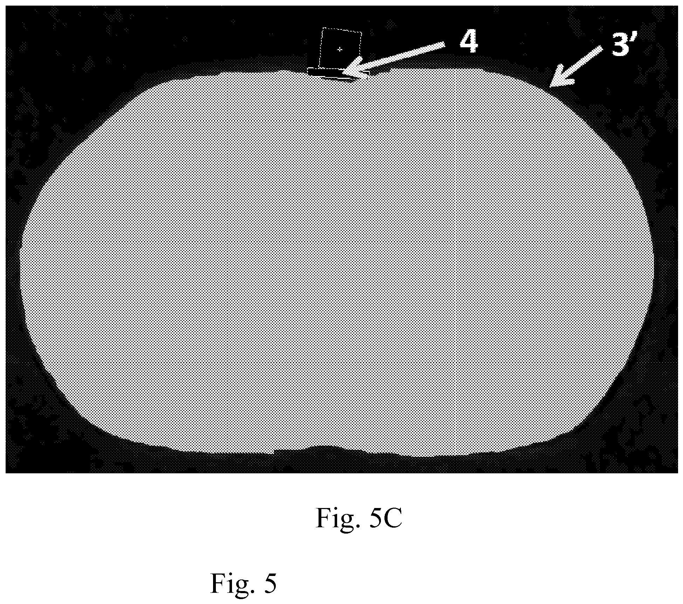

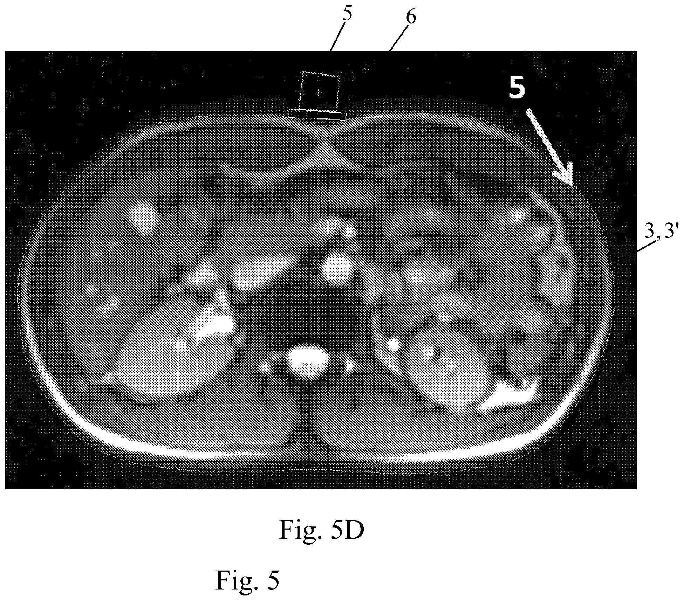

Cette segmentation axiale peut être réalisée en exécutant les quatre étapes opératoires suivantes (voir Fig. 5A à 5D) :
1) Un masque binaire correspondant au seuil de l'image originelle est calculé.
2) On ne conserve que les plus grands éléments connectés du masque et on remplit ce dernier (Fig. 5B et 5C).
3) La courbe correspondant à la limite périphérique du masque est extraite. Puis cette courbe est lissée et complétée pour pallier les erreurs et manques d'information dans l'image originelle.
4) La courbe correspondant à la position de la peau est corrigée à proximité du marqueur 5, connaissant la distance entre le centre du marqueur et la peau.

Comme déjà mentionné précédemment, et afin de tenir compte de l'hystérésis affectant la peau entre les phases d'inspiration et d'expiration, il est recommandé selon l'invention de construire un modèle de simulation différent de la peau en fonction de la phase du cycle respiratoire (inspiration et expiration).

A cet effet, les courbes illustrant les positions du marqueur 5 doivent être classées en deux catégories relatives l'une à la phase d'inspiration et l'autre à la phase d'expiration.

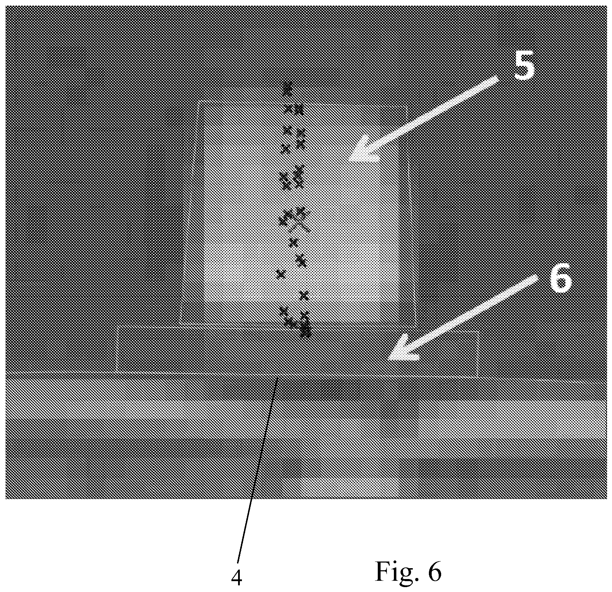

L'effet d'hystérésis est maximal à la fin de la phase d'inspiration et au début de la phase d'expiration, et la discrimination des courbes est triviale à l'exception de l'état d'inspiration extrême (voir Fig. 6).

Afin de pouvoir discriminer les positions du marqueur dans cet état critique, on peut avantageusement exploiter simultanément la position du centre du marqueur et la courbure de la peau autour du marqueur (les inventeurs ont constaté que la position latérale du marqueur changeait du fait de la modification de la courbure de la peau).

Dans le cadre d'une phase d'acquisition et de traitement préalable du procédé selon l'invention, les courbes représentant le profil 3 de la peau 2 dans des plans axiaux Pa espacés prédéterminés et à différents instants des phases d'inspiration et d'expiration peuvent être obtenues de différentes façons.

Ainsi, ces courbes peuvent être :
- extraites, par échantillonnage selon ces plans axiaux, de représentations tridimensionnelles de la surface de la peau 2 au niveau de la zone d'intérêt, acquises préalablement et obtenues par repérage et suivi de la peau au moyen de marqueurs (électromagnétiques, optiques ou autres) fixés sur la peau ou par l'intermédiaire de lumière structurée projetée sur la peau ;
- extraites, par application d'une technique de segmentation, de représentations tridimensionnelles obtenues par un procédé d'imagerie basé sur la résonnance magnétique ou la tomodensitométrie ;
- extraites, par segmentation, d'images du type 2D + t effectuées dans lesdits plans axiaux Pa et obtenues par un procédé d'imagerie basé sur la résonnance magnétique ou la tomodensitométrie.

Dans le cadre d'une application médicale avantageuse des résultats fournis par l'invention, la représentation tridimensionnelle évolutive 2' de la peau 2 au niveau de la zone d'intérêt est exploitée, en relation avec au moins une image tridimensionnelle de l'abdomen, pour réaliser une simulation prédictive en temps-réel des positions des organes et/ou viscères de la cavité abdominale durant des phases de respiration libre normale du sujet 1, en mettant par exemple en oeuvre l'enseignement de la publication citée dans la partie introductive de la présente description.

Ainsi, grâce à l'invention, il est possible de mettre à disposition une simulation tridimensionnelle évoluant en temps réel de la peau d'un patient, en ne relevant de manière courante que la position d'un nombre limité de points de la peau, donc en limitant les ressources de traitement nécessaires, et limitant la zone repérée nécessaire tout en fournissant une précision suffisante pour des applications médicales.

Une mise en oeuvre pratique de l'invention a permis d'atteindre une précision de 1 mm et un rafraîchissement de la simulation à une fréquence de 80 Hz.

De plus, par des évaluations comparatives, les inventeurs ont pu estimer que la simulation produite par l'invention présentait une précision au moins équivalente à celle d'une reconstruction de la peau directement à partir d'images acquises par le système d'imagerie.

L'invention porte également sur un système d'imagerie, notamment du type IRM, intégrant les moyens techniques matériels et logiciels autorisant la mise en oeuvre du procédé selon l'invention, ces moyens découlant de manière évidente de la description du procédé ci-dessus. Ce système peut, par exemple, comprendre un dispositif d'imagerie par résonance magnétique du type MAGNETOM Aera 1.5 Tesla de la société SIEMENS.

Bien entendu, l'invention n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention

## Revendications

1. Procédé automatique de détermination prédictive mis en oeuvre par un logiciel, sous la forme d'une simulation numérique, de la position et des déplacements de la peau d'un sujet humain ou animal dans une zone d'intérêt localisée au niveau du thorax et/ou de l'abdomen, ledit sujet étant préférentiellement allongé sur son dos et respirant librement ou de manière assistée,
ledit procédé consistant, de manière préalable, à acquérir ou déterminer plusieurs positions et configurations du profil de la peau dans des plans axiaux consécutifs prédéterminés, sensiblement perpendiculaires à l'axe crânio-caudal et répartis le long de cet axe à des emplacements déterminés, ceci à des instants successifs donnés, dans des positions respiratoires différentes, avantageusement durant des phases d'inspiration et d'expiration du sujet,
**ce** procédé **consistant également,**
- durant une phase de traitement préalable, à construire au moins un modèle numérique déformable indépendant du profil de la peau dans chaque plan axial (Pa), ce à partir d'informations relatives aux différents profils de peau (3) acquis ou déterminés précédemment,
- à relever ensuite, de manière répétitive, la position réelle d'un point ou d'une zone ponctuelle (4) spécifique de la peau (2) du sujet (1) au niveau de chacun des plans axiaux (Pa) précités, dont la position est modifiée de manière significative durant les phases d'inspiration et d'expiration, et
- à fournir, sensiblement en temps réel, d'une part, une simulation (3') du profil de la peau dans chaque plan axial (Pa), en fonction de la position réelle relevée pour chaque point ou zone ponctuelle (4) spécifique et en exploitant le modèle numérique déformable du profil de peau dans le plan (Pa) correspondant, et, d'autre part, une représentation tridimensionnelle (2') évolutive de la peau (2) au niveau de la zone d'intérêt, ce sur la base d'un modèle tridimensionnel de simulation globale de la peau composée de la pluralité de modèles axiaux bidimensionnels élémentaires, déformables et indépendants établis dans les plans axiaux (Pa) et par interpolation entre lesdits différents plans axiaux (Pa) précités,
procédé **caractérisé en ce que** chaque modèle numérique déformable de profil de peau, associé respectivement à l'un des plans axiaux (Pa) prédéterminés, est obtenu par appariement de points selon une direction sensiblement radiale, dans le plan axial (Pa) considéré, entre au moins deux conformations ou configurations sensiblement différentes de la courbe du profil de peau, correspondant à au moins deux états respectifs différents du mouvement respiratoire du sujet.

2. Procédé selon la revendication 1, **caractérisé en ce que** les points ou zones ponctuelles (4) spécifiques de la peau (2), dont la position réelle est suivie dans le temps, sont disposé(e)s dans un plan parallèle proche du ou confondu avec le plan sagittal médian (PSM) du sujet (1), en étant préférentiellement espacé(e)s mutuellement d'une distance de l'ordre du centimètre.

3. Procédé selon la revendication 1, **caractérisé en ce que** les points ou zones ponctuelles (4) spécifiques, dont la position réelle est suivie dans le temps, sont réparti(e)s le long de l'axe crânio-caudal, en étant aligné(e)s ou non, leur nombre étant avantageusement au moins égal à environ 10 et leur espacement d'au plus 5 cm dans la direction de l'axe précité.

4. Procédé selon l'une quelconque des revendications 1 à 3, **caractérisé en ce qu'**il consiste, pour chaque plan axial (Pa) concerné, à générer sensiblement en temps réel, une courbe (3') représentant le profil (3) de la peau (2) dans le plan axial (Pa) considéré, en appliquant à une courbe de base (Cb) de profil de peau, avantageusement déterminée dans un état extrême d'inspiration ou d'expiration du sujet, une déformation, par exemple par déplacement d'au moins certains points espacés de la courbe de base, fonction de la position mesurée d'un point (4) de la peau (2) du sujet (1) situé dans le plan axial (Pa) considéré.

5. Procédé selon l'une quelconque des revendications 1 à 4, **caractérisé en ce que** chaque modèle numérique déformable de profil de peau, associé à l'un des plans axiaux (Pa) prédéterminés, est obtenu par génération d'une courbe de référence (Cr) située à mi-distance entre les deux courbes de profils de peau correspondant aux états extrêmes d'inspiration et d'expiration, à déterminer les points d'intersection, avec les deux courbes précitées, d'une droite normale à la courbe de référence (Cr) et passant par un point de cette courbe, ce pour une pluralité de points espacés le long de cette dernière, et à rassembler de manière ordonnée ces couples de points d'intersection pour constituer un champ de déformation correspondant au déplacement de la peau (2) dans le plan axial (Pa) considéré entre les deux états extrêmes précités, en fonction de l'état respiratoire du sujet (1).

6. Procédé selon l'une quelconque des revendications 1 à 5, **caractérisé en ce qu'**il consiste à construire deux modèles numériques déformables de profil de peau pour chaque plan axial (Pa), à savoir un modèle pour les phases d'inspiration et un modèle pour les phases d'expiration, et à détecter la phase respiratoire en cours lors de l'élaboration des courbes simulées (3') de profil de peau dans les différents plans axiaux (Pa), ce par l'intermédiaire d'un capteur spécifique ou en évaluant le sens de déplacement des points (4).

7. Procédé selon l'une quelconque des revendications 1 à 6, **caractérisé en ce que** les positions des points (4) spécifiques de la peau (2) sont relevés en temps réel au moyen d'un dispositif capteur optique, notamment à laser, avec mise en place préalable ou non de marqueurs optiques sur la peau (2) du patient aux emplacements des points (4) spécifiques, ces derniers étant avantageusement sélectionnés de manière à obtenir une modification significative de leur position spatiale durant le mouvement respiratoire du sujet.

8. Procédé selon l'une quelconque des revendications 1 à 6, **caractérisé en ce que** les positions des points (4) de la peau (2) situés dans le plan sagittal médian (PSM) du sujet (1) et dans les plans axiaux (Pa) prédéterminés sont déterminées par l'intermédiaire d'au moins un marqueur (5).

9. Procédé selon la revendication 8, **caractérisé en ce que** les points (4) spécifiques de la peau (2) sont chacun repérés par l'intermédiaire d'un marqueur (5) particulièrement bien visible dans le procédé d'imagerie mis en oeuvre, ces points spécifiques (4) étant avantageusement localisés de telle manière que leur position spatiale est modifiée de manière significative durant le mouvement respiratoire du sujet.

10. Procédé selon la revendication 8 ou 9, **caractérisé en ce que** les marqueurs (5) mis en oeuvre consistent en des marqueurs de type électromagnétique, tels que par exemple des bobines miniatures, reposant directement ou indirectement sur la peau (2), le cas échéant avec un espacement connu par rapport à cette dernière.

11. Procédé selon la revendication 8 ou 9, **caractérisé en ce que** le marqueur (5) unique mis en oeuvre consiste en un marqueur allongé continu, reposant directement ou indirectement sur la peau (2) du patient (1), épousant la surface locale de la peau (2) et disposé dans le plan sagittal médian (PSM).

12. Procédé selon la revendication 11, **caractérisé en ce que** le marqueur (5) mis en oeuvre consiste en un corps souple et allongé, réalisé en un matériau particulièrement bien visible dans le système d'imagerie utilisé, s'étendant au moins sur la longueur de la zone d'intérêt dans la direction de l'axe crânio-caudal (X), présentant une section de forme et de dimension minimale déterminées et situé à une distance déterminée de la peau (2) du sujet (1), en reposant sur une bande (6) d'un matériau souple et sensiblement non visible dans le système d'imagerie utilisé.

13. Procédé selon l'une quelconque des revendications 11 et 12, **caractérisé en ce qu'**il consiste à déterminer et à relever, dans le plan sagittal, la courbe (Cm) correspondant à l'axe médian du marqueur (5) allongé, par exemple formé par la succession des différents centres de gravité en section dudit marqueur (5), et/ou dans les différents plans axiaux (Pa) concernés, les centres de gravité locaux dudit marqueur (5), et à déterminer les positions des points spécifiques (4) de la peau (2) du sujet (1) dont les mouvements sont pris en compte, en exploitant les informations relatives, d'une part, à cette courbe (Cm) ou à ces centres de gravité locaux, d'autre part, à la section et aux dimensions en section du marqueur (5) et, enfin, à l'épaisseur de la bande de matériau (6) intercalaire.

14. Procédé selon l'une quelconque des revendications 1 à 13, **caractérisé en ce que** les courbes représentant le profil (3) de la peau (2) dans des plans axiaux (Pa) espacés prédéterminés et à différents instants des phases d'inspiration et d'expiration sont extraites, par échantillonnage selon ces plans axiaux, de représentations tridimensionnelles de la surface de la peau (2) au niveau de la zone d'intérêt, acquises préalablement et obtenues par repérage et suivi de la peau au moyen de marqueurs fixés sur la peau ou par l'intermédiaire de lumière structurée projetée sur la peau.

15. Procédé selon l'une quelconque des revendications 1 à 14, **caractérisé en ce que** les courbes représentant le profil (3) de la peau (2) dans des plans axiaux (Pa) espacés prédéterminés et à différents instants des phases d'inspiration et d'expiration sont extraites, par application d'une technique de segmentation, de représentations tridimensionnelles obtenues par un procédé d'imagerie basé sur la résonnance magnétique ou la tomodensitométrie.

16. Procédé selon l'une quelconque des revendications 1 à 14, **caractérisé en ce que** les courbes représentant le profil (3) de la peau (2) dans des plans axiaux (Pa) espacés prédéterminés et à différents instants des phases d'inspiration et d'expiration sont extraites, par segmentation, d'images du type 2D + t effectuées dans lesdits plans axiaux (Pa) et obtenues par un procédé d'imagerie basé sur la résonnance magnétique ou la tomodensitométrie.

17. Procédé selon l'une quelconque des revendications 1 à 15, **caractérisé en ce que** la représentation tridimensionnelle évolutive de la peau (2) au niveau de la zone d'intérêt est exploitée, en relation avec au moins une image tridimensionnelle de l'abdomen, pour réaliser une simulation prédictive en temps réel des positions des organes et/ou viscères de la cavité abdominale durant des phases de respiration libre normale du sujet (1).

18. Procédé selon l'une quelconque des revendications 11 à 13, **caractérisé en ce que**, lorsque le procédé d'imagerie auquel est soumis le sujet (1) est basé sur la résonance magnétique, le marqueur allongé (5) est constitué par une barre formée d'eau gélifiée, avec une section carrée ou rectangulaire et une longueur suffisante pour couvrir au moins la zone du sujet (1) dont on souhaite suivre les mouvements induits par la respiration dudit sujet.

## Patentansprüche

1. Automatisches Verfahren zur prädiktiven Bestimmung, ausgeführt durch ein Computerprogramm, in Form einer digitalen Simulation der Position und der Bewegungen der Haut eines menschlichen oder tierischen Individuums in einem interessierenden Bereich der Brust und/oder des Bauches, wobei das genannte Individuum vorzugsweise auf dem Rücken liegt und frei oder assistiert atmet, wobei das genannte Verfahren darin besteht, vorab mehrere Positionen und Konfigurationen des Hautprofils in festgelegten, axial aufeinanderfolgenden Ebenen zu erfassen oder zu bestimmen, die zur craniocaudalen Achse im Wesentlichen rechtwinklig sind und über diese Achse an bestimmten Stellen verteilt sind, dies zu gegebenen, aufeinanderfolgenden Zeitpunkten in verschiedenen Atmungspositionen, vorteilhafterweise während Inspirations- und Exspirationsphasen des Individuums,
wobei dieses Verfahren ebenfalls darin besteht,
- in einer Verarbeitungsphase vorab mindestens ein unabhängiges verformbares, digitales Modell des Hautprofils in jeder axialen Ebene (Pa) zu konstruieren, dies aus Informationen über die verschiedenen Hautprofile (3), die vorab erfasst oder bestimmt wurden,
- anschließend wiederholt die tatsächliche Position eines bestimmten Punktes oder punktförmigen Bereiches (4) der Haut (2) des Individuums (1) in Höhe jeder der genannten axialen Ebenen (Pa) zu erfassen, dessen Position während der Inspirations- und Exspirationsphasen erheblich geändert wird, und
- im Wesentlichen in Echtzeit einerseits eine Simulation (3') des Hautprofils in jeder axialen Ebene (Pa) in Abhängigkeit von der für jeden bestimmten Punkt oder punktförmigen Bereich (4) erfassten tatsächlichen Position und unter Verwendung des verformbaren digitalen Modells des Hautprofils in der entsprechenden Ebene (Pa) auszugeben und andererseits eine evolutive, dreidimensionale Darstellung (2') der Haut (2) im interessierenden Bereich auszugeben, dies auf der Grundlage eines globalen dreidimensionalen Simulationsmodells der Haut, bestehend aus den mehreren zweidimensionalen, axialen, verformbaren und unabhängigen Elementarmodellen, die in den axialen Ebenen (Pa) erstellt wurden, und durch Interpolation zwischen den verschiedenen genannten axialen Ebenen (Pa), Verfahren, **dadurch gekennzeichnet, dass** jedes verformbare digitale Hautprofilmodell, das jeweils einer der vorab festgelegten axialen Ebenen (Pa) entspricht, durch Punktepaarung in einer im Wesentlichen radialen Richtung in der betrachteten axialen Ebene (Pa) zwischen mindestens zwei deutlich unterschiedlichen Formen oder Konfigurationen der Kurve des Hautprofils, die mindestens zwei unterschiedlichen, entsprechenden Zuständen der Atmungsbewegungen des Individuums entsprechen, erhalten wird.

2. Verfahren nach Patentanspruch 1, **dadurch gekennzeichnet, dass** die bestimmten Punkte oder punktförmigen Bereiche (4) der Haut (2), deren tatsächliche Position in der Zeit verfolgt wird, in einer parallelen Ebene liegen, die der Sagittal-Medianebene (PSM) des Individuums (1) nahe oder mit ihr identisch ist, während sie vorzugsweise voneinander um einen Abstand in der Größenordnung von Zentimetern beabstandet sind.

3. Verfahren nach Patentanspruch 1, **dadurch gekennzeichnet, dass** die bestimmten Punkte oder punktförmigen Bereiche (4), deren tatsächliche Position in der Zeit verfolgt wird, über die craniocaudale Achse verteilt sind, ausgerichtet oder nicht, wobei ihre Anzahl vorteilhafterweise mindestens gleich ungefähr 10 ist und ihr Abstand höchstens 5 cm in der Richtung der genannten Achse beträgt.

4. Verfahren nach irgendeinem der Patentansprüche 1 bis 3, **dadurch gekennzeichnet, dass** es darin besteht, für jede betroffene axiale Ebene (Pa) im Wesentlichen in Echtzeit eine Kurve (3') zu erzeugen, die das Profil (3) der Haut (2) in der betrachteten axialen Ebene (Pa) darstellt, indem auf eine Basishautprofilkurve (Cb), die vorteilhafterweise in einem Zustand extremer Inspiration oder Exspiration des Individuums ermittelt wurde, eine Verformung ausgeübt wird, beispielsweise durch Versetzen mindestens bestimmter, voneinander beabstandeter Punkte aus der Basiskurve in Abhängigkeit von der gemessenen Position eines Punktes (4) der Haut (2) des Individuums (1), der sich in der betrachteten axialen Ebene (Pa) befindet.

5. Verfahren nach irgendeinem der Patentansprüche 1 bis 4, **dadurch gekennzeichnet, dass** jedes digitale, verformbare Hautprofilmodell, das einer der vorab festgelegten axialen Ebenen (Pa) zugehört, durch Bestimmung einer Bezugskurve (Cr) erhalten wird, die sich in der Mitte zwischen den beiden Hautprofilkurven befindet, die den extremen Inspirations- und Exspirationszuständen entsprechen, die Schnittpunkte einer zur Bezugskurve (Cr) normalen und durch einen Punkt dieser Kurve verlaufende Gerade mit den beiden genannten Kurven für mehrere auf dieser liegende, voneinander beabstandete Punkte zu bestimmen und in geordneter Weise diese Schnittpunktpaare zu vereinen, um ein Verformungsfeld zu bilden, das der Verformung der Haut (2) in der betrachteten axialen Ebene (Pa) zwischen den beiden genannten Extremzuständen in Abhängigkeit vom Atmungszustand des Individuums (1) entspricht.

6. Verfahren nach irgendeinem der Patentansprüche 1 bis 5, **dadurch gekennzeichnet, dass** es darin besteht, zwei digitale, verformbare Hautprofilmodelle für jede axiale Ebene (Pa) zu konstruieren, nämlich ein Modell für die Inspirationsphasen und ein Modell für die Exspirationsphasen, und die laufende Atmungsphase während der Bestimmung der simulierten Hautprofilkurven (3') in den verschiedenen axialen Ebenen (Pa) mit Hilfe eines spezifischen Sensors oder durch Auswertung der Verschiebungsrichtung der Punkte (4) festzustellen.

7. Verfahren nach irgendeinem der Patentansprüche 1 bis 6, **dadurch gekennzeichnet, dass** die Positionen der spezifischen Punkte (4) der Haut (2) in Echtzeit mit Hilfe einer optischen Sensorvorrichtung, insbesondere mit einem Laser, unter vorheriger Anordnung optischer Markierungen auf der Haut des Patienten an den Stellen der spezifischen Punkte (4) oder nicht, erfasst werden, wobei diese letztgenannten vorteilhafterweise derart gewählt werden, dass eine deutliche Änderung ihrer räumlichen Position während der Atembewegung des Individuums erhalten wird.

8. Verfahren nach irgendeinem der Patentansprüche 1 bis 6, **dadurch gekennzeichnet, dass** die Positionen der Punkte (4) der Haut (2), die in der Sagittal-Medianebene (PSM) des Individuums (1) und in den vorab festgelegten axialen Ebenen (Pa) liegen, mit Hilfe mindestens einer Markierung (5) bestimmt werden.

9. Verfahren nach Patentanspruch 8, **dadurch gekennzeichnet, dass** die spezifischen Punkte (4) der Haut (2) jeweils mit Hilfe einer im angewandten bildgebenden Verfahren gut sichtbaren Markierung (5) bestimmt werden, wobei diese spezifischen Punkte (4) vorteilhafterweise derart angeordnet sind, dass ihre räumliche Position sich während der Atembewegung des Individuums deutlich ändert.

10. Verfahren nach Patentanspruch 8 oder 9, **dadurch gekennzeichnet, dass** die verwendeten Markierungen (5) Markierungen vom elektromagnetischen Typ sind, wie etwa Miniaturspulen, die unmittelbar oder mittelbar auf der Haut (2) ruhen, gegebenenfalls in einem bekannten Abstand von dieser.

11. Verfahren nach Patentanspruch 8 oder 9, **dadurch gekennzeichnet, dass** die einzige verwendete Markierung (5) in einer durchgehenden, langgestreckten Markierung besteht, die unmittelbar oder mittelbar auf der Haut (2) des Patienten (1) ruht, indem sie sich der lokalen Oberfläche der Haut (2) anschmiegt und in der Sagittal-Medianebene (PSM) angeordnet ist.

12. Verfahren nach Patentanspruch 11, **dadurch gekennzeichnet, dass** die verwendete Markierung (5) in einem weichen und langgestreckten Körper besteht, der aus einem Werkstoff gefertigt ist, der im verwendeten bildgebenden Verfahren besonders gut sichtbar ist, sich mindestens über die Länge des interessierenden Bereiches in Richtung der craniocaudalen Achse (X) erstreckt und einen festgelegten Querschnitt mit festgelegter kleinster Abmessung aufweist und in einem festgelegten Abstand von der Haut (2) des Individuums (1) angeordnet ist, wobei er auf einem Streifen (6) aus weichem und im verwendeten bildgebenden Verfahren im Wesentlichen nicht sichtbaren Werkstoff besteht.

13. Verfahren nach irgendeinem der Patentansprüche 11 und 12, **dadurch gekennzeichnet, dass** es darin besteht, in der Sagittalebene die Kurve (Cm) zu bestimmen und zu erfassen, die der Mittelachse der langgestreckten Markierung (5) entspricht, die beispielsweise durch die Reihe der verschiedenen Schwerpunkte des Querschnitts der genannten Markierung (5) und/oder die lokalen Schwerpunkte der Markierung (5) in den verschiedenen betroffenen axialen Ebenen (Pa) gebildet wird, und die Stellen der spezifischen Punkte (4) der Haut (2) des Individuums (1) zu bestimmen, deren Bewegungen berücksichtigt werden, indem die Informationen über einerseits diese Kurve (Cm) oder diese lokalen Schwerpunkte und andererseits über den Querschnitt und die Querschnittsabmessungen der Markierung (5), und schließlich über die Dicke des eingefügten Werkstoffstreifens (6) ausgewertet werden.

14. Verfahren nach irgendeinem der Patentansprüche 1 bis 13, **dadurch gekennzeichnet, dass** die Kurven, die das Profil (3) der Haut (2) in den vorab festgelegten, voneinander beabstandeten axialen Ebenen (Pa) und zu verschiedenen Zeitpunkten der Inspirations- und Exspirationsphasen darstellen, durch Abtastung dreidimensionaler Darstellungen der Oberfläche der Haut (2), die im interessierenden Bereich, die durch Kennzeichnung und Verfolgen der Haut mit Hilfe auf der Haut befestigter Markierungen oder durch strukturiertes Licht, das auf die Haut projiziert wurde, vorab erfasst wurden, nach axialen Ebenen gewonnen werden.

15. Verfahren nach irgendeinem der Patentansprüche 1 bis 14, **dadurch gekennzeichnet, dass** die Kurven, die das Profil (3) der Haut (2) in vorab festgelegten, voneinander beabstandeten axialen Ebenen (Pa) und zu verschiedenen Zeitpunkten der Inspirations- und Exspirationsphasen darstellen, durch eine Technik der Segmentierung dreidimensionaler Darstellungen gewonnen werden, die durch ein bildgebendes Verfahren auf der Grundlage der Magnetspinresonanz oder der Computertomographie erhalten wurden.

16. Verfahren nach irgendeinem der Patentansprüche 1 bis 14, **dadurch gekennzeichnet, dass** die Kurven, die das Profil (3) der Haut (2) in den vorab festgelegten, voneinander beabstandeten axialen Ebenen (Pa) und zu verschiedenen Zeitpunkten der Inspirations- und Exspirationsphasen darstellen, durch Segmentierung von Bildern des Typs 2D + t gewonnen werden, die in den genannten Achsenebenen (Pa) erfolgt, und durch ein bildgebendes Verfahren auf der Grundlage der Magnetspinresonanz oder der Computertomographie erhalten wurden.

17. Verfahren nach irgendeinem der Patentansprüche 1 bis 15, **dadurch gekennzeichnet, dass** die evolutive dreidimensionale Darstellung der Haut (2) im interessierenden Bereich in Verbindung mit mindestens einem dreidimensionalen Bild des Bauches ausgewertet wird, um eine vorhersagende Simulation in Echtzeit der Stellungen der Organe und/oder Eingeweide der Bauchhöhle während der Phasen normaler, freier Atmung des Individuums (1) zu erstellen.

18. Verfahren nach irgendeinem der Patentansprüche 11 bis 13, **dadurch gekennzeichnet, dass** die langgestreckte Markierung (5), wenn das bildgebende Verfahren, dem das Individuum unterworfen wird, auf Magnetspinresonanz beruht, aus einem Stab aus geliertem Wasser mit quadratischem oder rechteckigem Querschnitt und einer Länge besteht, die ausreicht, um mindestens den Bereich des Individuums (1) abzudecken, dessen durch die Atmung des Individuums bewirkte Bewegungen verfolgt werden sollen.

## Claims

1. Automatic process of predictive determination implemented by software, in the form of a digital simulation, of the position and movements of the skin of a human subject or an animal in an area of interest located at the level of the thorax and/or the abdomen, with said subject preferably being stretched out on their back and breathing freely or in an assisted manner,
said process comprising, in a preliminary manner, acquiring or determining multiple positions and configurations of the profile of the skin in predetermined, consecutive, axial planes that are essentially perpendicular to the craniocaudal axis and distributed along this axis at determined locations, at given successive times, in different respiratory positions, advantageously during inhalation and exhalation phases of the subject,
the process further comprising,
- during a preliminary treatment phase, constructing at least one independent deformable digital model of the profile of the skin in each axial plane (Pa) from data pertaining to different skin profiles (3) that are acquired or determined previously,
- then noting, in a repetitive manner, the actual position of a point or a specific point area (4) on the skin (2) of the subject (1) at the level of each of the aforementioned axial planes (Pa), whose position is modified in a significant manner during the inhalation and exhalation phases, and
- providing, essentially in real time, on the one hand, a simulation (3') of the profile of the skin in each axial plane (Pa), as a function of the actual position noted for each point or specific point area (4) and by exploiting the deformable digital model of the skin profile in the corresponding plane (Pa), and, on the other hand, an evolving three-dimensional representation (2') of the skin (2) at the level of the area of interest, this on the basis of a three-dimensional model for overall simulation of the skin composed of the plurality of deformable and independent elementary two-dimensional axial models established in the axial planes (Pa) and by interpolation between said different aforementioned axial planes (Pa),
the process being **characterised in that** each deformable digital model of a skin profile, respectively associated with one of the predetermined axial planes (Pa), is obtained by pairing points in an essentially radial direction, in the axial plane (Pa) in question, between at least two shapes or configurations that are significantly different from the curve of the skin profile, corresponding to at least two different respective states of the respiratory movement of the subject.

2. Process according to claim 1, **characterised in that** the points or specific point areas (4) of the skin (2), whose actual position is monitored over time, are placed in a parallel plane that is close to or merged with the mid-sagittal plane (MSP) of the subject (1), by preferably being spaced apart mutually by a distance on the order of one centimetre.

3. Process according to claim 1, **characterised in that** the points or specific point areas (4), whose actual position is monitored over time, are distributed along the craniocaudal axis by being aligned or not, with their number advantageously being at least equal to approximately 10 and their spacing at most 5 cm in the direction of the aforementioned axis.

4. Process according to any of claims 1 to 3, **characterised in that** it consists, for each axial plane (Pa) in question, of generating essentially in real time a curve (3') that represents the profile (3) of the skin (2) in the axial plane (Pa) in question by applying a deformation to a base curve (Cb) of the skin profile, advantageously determined in an end inhalation or exhalation state of the subject, for example by movement of at least certain points spaced on the base curve, based on the measured position of a point (4) on the skin (2) of the subject (1) located in the axial plane (Pa) in question.

5. Process according to any of claims 1 to 4, **characterised in that** each deformable digital model of a skin profile, associated with one of the predetermined axial planes (Pa), is obtained by generating a reference curve (Cr), located mid-way between the two skin profile curves corresponding to the end inhalation and exhalation states, to determine the intersection points, with the two aforementioned curves, of a normal to the reference curve (Cr) and passing through a point of this curve, for a number of points spaced along the latter, and to assemble in an ordered manner these pairs of intersection points to form a deformation field corresponding to the movement of the skin (2) in the axial plane (Pa) in question between the two aforementioned end states, as a function of the respiratory state of the subject (1).

6. Process according to any of claims 1 to 5, **characterised in that** it consists of constructing two deformable digital models of a skin profile for each axial plane (Pa), namely a model for the inhalation phases and a model for the exhalation phases, and detecting the respiratory phase in progress during the production of simulated curves (3') of a skin profile in the different axial planes (Pa) by means of a specific sensor or by evaluating the direction of movement of the points (4).

7. Process according to any of claims 1 to 6, **characterised in that** the specific point positions (4) on the skin (2) are noted in real time by means of an optical sensor device, in particular with a laser, with or without preliminary installation of optical markers on the patient's skin (2) in the locations of specific points (4), with the latter advantageously being selected in such a way as to obtain a significant modification of their spatial position during the respiratory movement of the subject.

8. Process according to any of claims 1 to 6, **characterised in that** the positions of the points (4) of the skin (2) located in the mid-sagittal plane (MSP) of the subject (1) and in the predetermined axial planes (Pa) are determined by means of at least one marker (5).

9. Process according to claim 8, **characterised in that** the specific points (4) on the skin (2) are each highlighted by means of a marker (5) that is particularly readily visible in the imagery process that is used, with these specific points (4) being advantageously located in such a way that their spatial position is modified significantly during the respiratory movement of the subject.

10. Process according to claim 8 or 9, **characterised in that** the markers (5) that are used consist of electromagnetic-type markers, such as, for example, miniature coils, resting directly or indirectly on the skin (2), if necessary with a known spacing relative to the latter.

11. Process according to claim 8 or 9, **characterised in that** the one marker (5) that is used consists of a continuously elongated marker, resting directly or indirectly on the skin (2) of the patient (1), conforming in shape to the local surface of the skin (2) and placed in the mid-sagittal plane (MSP).

12. Process according to claim 11, **characterised in that** the marker (5) that is used consists of a flexible and elongated body, made of a material that is particularly readily visible in the imagery system used, extending at least over the length of the area of interest in the direction of the craniocaudal axis (X), exhibiting a cross-section of determined minimal size and shape and located at a determined distance from the skin (2) of the subject (1), by resting on a strip (6) of a material that is flexible and essentially invisible in the imagery system that is used.

13. Process according to any of claims 11 and 12, **characterised in that** it consist of determining and noting, in the sagittal plane, the curve (Cm) corresponding to the median axis of the elongated marker (5), for example formed by the series of different centres of gravity in the cross-section of said marker (5), and/or in the different axial planes (Pa) in question, the local centres of gravity of said marker (5), and determining the positions of specific points (4) on the skin (2) of the subject (1) whose movements are taken into account, by exploiting the data pertaining, on the one hand, to this curve (Cm) or to these local centres of gravity, on the other hand, to the cross-section and to the cross-sectional dimensions of the marker (5), and finally, to the thickness of the intermediate material strip (6).

14. Process according to any of claims 1 to 13, **characterised in that** the curves showing the profile (3) of the skin (2) in spaced axial planes (Pa) that are predetermined and at different times for the inhalation and exhalation phases are extracted by sampling along these axial planes from three-dimensional representations of the surface of the skin (2) at the level of the area of interest, acquired in advance and obtained by highlighting and monitoring the skin by means of markers attached to the skin or by means of structured light projected on the skin.

15. Process according to any of claims 1 to 14, **characterised in that** the curves showing the profile (3) of the skin (2) in spaced axial planes (Pa) that are predetermined and at different times for the inhalation and exhalation phases are extracted, by application of a segmentation technique, from three-dimensional representations obtained by an imagery process based on magnetic resonance or tomodensitometry.

16. Process according to any of claims 1 to 14, **characterised in that** the curves showing the profile (3) of the skin (2) in spaced axial planes (Pa) that are predetermined and at different times for the inhalation and exhalation phases are extracted, by segmentation, from 2D+t-type images made in said axial planes (Pa) and obtained by an imagery process based on magnetic resonance or tomodensitometry.

17. Process according to any of claims 1 to 15, **characterised in that** the evolving three-dimensional representation of the skin (2) at the level of the area of interest is exploited, in connection with at least one three-dimensional image of the abdomen, for carrying out a predictive simulation in real time of the positions of the organs and/or viscera of the abdominal cavity during normal free breathing phases of the subject (1).

18. Process according to any of claims 11 to 13, **characterised in that** when the imagery process to which the subject (1) is subjected is based on magnetic resonance, the elongated marker (5) consists of a bar formed by water gel, with a square or rectangular cross-section, and a sufficient length to cover at least the area of the subject (1) whose movements induced by the breathing of said subject are to be monitored.
